# EUROPÄISCHE PATENTSCHRIFT

(11) **EP 3 391 018 B1**
(45) Veröffentlichungstag und Bekanntmachung des Hinweises auf die Patenterteilung: **28.07.2021**
(21) Anmeldenummer: 16819846.3
(22) Anmeldetag: 14.12.2016
(51) Int. Cl.: G01N 3/00, D07B 1/14

(54) **VERFAHREN ZUR BESTIMMUNG DER ABLEGEREIFE EINES SEILES AUS TEXTILEM FASERMATERIAL**
METHOD FOR DETERMINING THE POINT OF DISCARD OF A ROPE MADE OF TEXTILE FIBROUS MATERIAL
PROCEDE DE DETERMINATION D'UN ETAT D'USURE NECESSITANT LE REMPLACEMENT D'UN CABLE EN MATERIAU TEXTILE FIBREUX

(30) Priorität: 16.12.2015 EP 15200442
(43) Veröffentlichungstag der Anmeldung: 24.10.2018
(73) Patentinhaber: Teufelberger Fiber Rope GmbH, 4600 Wels (AT)
(72) Erfinder: ERNST, Björn, A-4810 Gmunden (AT); RÜHRNÖSSL, Erich, 4053 Haid (AT); KIRTH, Rudolf, 4840 Vöcklabruck (AT); BALDINGER, Peter, 4311 Schwertberg (AT); TRAXL, Robert, 4802 Ebensee (AT); KAISER, Gunter, 4600 Thalheim/Wels (AT)
(74) Vertreter: Nemec, Harald
(86) Internationale Anmeldenummer: PCT/EP2016/080957
(87) Internationale Veröffentlichungsnummer: WO 2017/102821

(56) Entgegenhaltungen:
- EP-A1- 1 530 040
- EP-A2- 1 905 892
- WO-A1-2004/029343
- WO-A1-2015/139842
- JP-A- 2001 192 183
- US-A1- 2003 111 298
- US-A1- 2005 226 584

## Beschreibung

Die vorliegende Erfindung betrifft ein Verfahren zur Bestimmung der Ablegereife eines Seiles aus textilem Fasermaterial.

Seile aus textilem Fasermaterial, zum Beispiel Kunstfaserseile, werden für zahlreiche Anwendungen eingesetzt. Besonders auf dem Gebiet der Fördertechnik sind hochfeste Faserseile den früher ausschließlich verwendeten bzw. verfügbaren Stahlseilen mittlerweile bereits aufgrund mehrerer Vorteile überlegen.

Seile werden im Zuge ihrer Verwendung verschiedenen, insbesondere mechanischen, Belastungen ausgesetzt. Unter "Ablegereife" ("point of discard") versteht der Fachmann jenen Punkt, an dem das Seil aufgrund des durch diese Belastungen verursachten Verschleisses nicht mehr bzw. nicht mehr in ausreichend sicherem Umfang weiter verwendet werden kann und daher außer Gebrauch genommen werden muss.

Die Feststellung der Ablegereife ist natürlich insbesondere bei Anwendungen wie z.B. Kranseilen oder auch Seilen in Aufzügen besonders wichtig.

Es sind verschiedene Methoden bekannt, die Ablegereife eines Seiles aus textilem Fasermaterial festzustellen.

Am Markt hat sich bisher keine dieser Methoden durchgesetzt. Die bisher fehlende Möglichkeit zur sicheren Erkennung der Ablegereife von Faserseilen ist als einer der Hauptgründe dafür zu sehen, dass sich Faserseile bisher im industriellen Einsatz noch nicht durchgesetzt haben.

Den meisten der veröffentlichten Methoden ist zu eigen, dass sie jeweils nur ein Charakteristikum des Seiles erfassen (z.B. Durchmesser, Längung, o.ä.). Da jedoch in den meisten Anwendungen regelmäßig mehrere verschiedene Schädigungsmechanismen wirken, die sich gegenseitig beeinflussen, sind solche Verfahren nicht hinreichend, um die Ablegereife eines Faserseiles sicher erkennen und vorhersagen zu können.

Es sind verschiedene Verfahren bekannt, bei denen in das Fasermaterial des Seiles, insbesondere in das lasttragende Fasermaterial, eine oder mehrere zusätzliche andersartige Fasern (zumeist mit Sensor- oder Übertragungsfunktion) in das Seil eingebaut werden, von deren Zustandsänderung auf die Zustandsänderung des kompletten Seilverbundes geschlossen können werden soll.

So beschreibt die EP 1 930 497 A die Verwendung einer Indikatorfaser, die eine gegenüber den übrigen Litzen des Seiles geringere Widerstandsfähigkeit gegen Abrieb aufweist.

Dieser Zugang ist problematisch, da die zugrundliegende Annahme, dass eine einzelne Indikatorfaser dieselbe Beanspruchung erfährt wie der höchstbeanspruchte Teil oder Abschnitt des Seiles, nicht immer zulässig ist.

Ebenfalls sind Verfahren bekannt, welche die Längung des Seiles über die Lebensdauer als Bewertungskriterium für den Zustand des Seiles sowie die Vorhersage der Ablegereife heranziehen und auf verschiedene Weise bestimmen, so z.B. aus der EP 0 731 209 A und der EP 2 002 051 A. In letzterem Dokument sind auf dem Mantel eines Kern-/Mantelseiles Markierungen (z.B. Flechtrauten aus unterschiedlich gefärbtem Material) vorgesehen, anhand derer man Längungen oder auch Verdrehungen des Seiles feststellen kann.

Aus der DE 20 2013 101 326 U1 ist die Verwendung eines elektrisch leitenden Sensorfadens bekannt. Die WO 2003/054290 A1 schlägt ein ferromagnetisches Material vor, mit welchem man auch lokale Schädigung des Seiles erkennen können soll.

Weiterer Stand der Technik ist z.B. aus der US 2003/111298 A1 sowie der US 2005/226584 A1 bekannt.

Faserseile für Hebeanwendungen und auf Winden (z.B. Hubseile) werden regelmäßig aus hochfesten Faserwerkstoffen hergestellt (zum Beispiel, aber nicht ausschließlich UHMWPE, Aramid, LCP und/oder PBO).

Bei Verwendung dieser Werkstoffe als Grundmaterial für die tragenden Elemente des Seiles (Litzen) sind die materialinhärenten Eigenschaften zu berücksichtigen.

So ist insbesondere das Kriechverhalten textiler Fasern von Relevanz.

Unter "Kriechen" versteht man die irreversible Längung des Materials bei Aufbringung einer geringeren Last als der Bruchlast des Seiles über eine Einwirkungsdauer bei einer gewissen Temperatur.

Dieses Kriechverhalten ist z.B. bei Fasern aus UHMWPE (z.B. Dyneema®) besonders ausgeprägt.

Im Folgenden wird daher insbesondere das Kriechverhalten von Fasern aus UHMWPE besprochen. Die Ausführungen gelten aber auch allgemein für andere (hochfeste oder auch nicht hochfeste) Fasertypen.

Die über UHMWPE publizierten Faserdaten zeigen klar, dass das Kriechen von UHMWPE-Fasern, wie sie in Seilen verbaut werden, von drei Faktoren abhängt:
1. der im Material wirkenden Zugspannung
2. der Temperatur
3. der Einwirkdauer (=Zeit)

Erhöhte Zugspannung, erhöhte Temperatur und verlängerte Einwirkdauer führen jede für sich zu einer erhöhten Längung des Materials. Hierbei ist zu beachten, dass die Abhängigkeit der Kriechrate (=Längung je Zeiteinheit) von Zugspannung und Temperatur nichtlinear ist.

Da die Seile im Einsatz regelmäßig nicht vorhersagbaren Anforderungen aus unterschiedlichen Zugspannungen, Temperaturen und Einsatzzeiten unterworfen sind, versagen sie oftmals nicht auf Grund von Kriechen als Versagensursache, sondern auf Grund anderer dominanter Schädigungsmechanismen.

Folglich weisen die Seile zum Zeitpunkt der Ablegereife bzw. zum Versagenszeitpunkt unterschiedliche Längungen auf.

Aber auch wenn Kriechen der Grund des Versagens ist, weisen die Seile zum Zeitpunkt der Ablegereife bzw. zum Versagenszeitpunkt unterschiedliche Längungen auf, sodass eine Erkennung der Ablegereife anhand der Längung des Seiles bisher nicht möglich war.

Es ist wissenschaftlich publiziert, dass die Längung von UHMWPE-Fasern, wenn sie den Bereich des tertiären Kriechens (= rapider Tragkraftverlust der Faser durch Brechen der Molekülketten) erreichen, nicht konstant ist, sondern stets abhängig von Last, Temperatur und Zeit. (Vlasblom/Bosman 2006: Predicting the Creep Lifetime of HMPE Mooring Rope Applications, Figur 17).

Daraus wurde der Schluss gezogen, dass die Längung des Seiles bis zur Ablegereife ebenfalls von Last, Temperatur und Zeit abhängig und nicht konstant ist.

Für stehende Seile, die im Betrieb nicht über Scheiben gebogen werden, wird im genannten Artikel von Vlasblom/Bosman unter gewissen Einschränkungen eine maximale zulässige Längung des Seiles über die gesamte Länge von 10% als Ablegekriterium angenommen. Diese maximal zulässige Längung des Seiles liegt weit unterhalb der minimalen Längung der Faser durch bei Bruch durch Kriechen.

Für laufende Seile, die im Betrieb über Scheiben gebogen werden, liegt eine solche Empfehlung nicht vor.

Auf Basis der Literaturdaten geht der Fachmann somit derzeit von folgenden Annahmen aus:
- Wenn Kriechen der Versagensmechanismus der Faser ist, so ist die Längung der Faser bis Bruch nicht konstant sondern stets abhängig von Last, Temperatur und Zeit.
- Wenn Kriechen der Versagensmechanismus des Seiles ist, so ist die Längung des Seiles bis Bruch nicht konstant, sondern stets abhängig von Last, Temperatur und Zeit.
- In Anwendungen mit nicht konstanter Last, Temperatur und Zeit ist die Längung von Faser bzw. Seil abhängig vom Lastkollektiv (also dem Last-, Temperatur- und Zeitverlauf).
- Die Beanspruchungshistorie der Faser/des Seiles beeinflusst somit die Längung der Faser / des Seiles bis Bruch.
- In laufenden Anwendungen lässt das Erreichen einer bestimmten absoluten Faser-/Seillängung somit keine zuverlässige Aussage über die Restlängung der Faser/des Seiles bis Bruch zu (da die Längung der Faser/des Seiles bis Bruch nicht konstant ist).
- Das Erreichen einer bestimmten absoluten Seillängung lässt analog keine zuverlässige Aussage über die Restlängung des Seiles bis Bruch und somit zur Bestimmung des Zeitpunktes der Ablegereife zu.
- Die Längung des Seiles bis Bruch ist von der Längung der Einzelfaser bis Bruch unterschiedlich. Dies liegt in der Verarbeitung der Fasern zum Seil (Zwirnen, verlitzen, flechten, drehen, u.a.) begründet. Siehe Vlasblom/Bosman 2006, Figur 14.

Zusätzlich stellt sich bei Seilen aus unterschiedlichen Fasermaterialien A bzw. B, die jeweils ein Kriechverhalten aufweisen, heraus, dass aus dem Verhalten (Längung/Zeitdauer) der Einzelfasern A bzw. B bei Bruch kein analoger Schluss auf das Verhalten von Seilen aus dem jeweiligen Fasermaterial A bzw. B bei Bruch gezogen werden kann. Zur Bestimmung der Längung eines Seiles bis Bruch müsste also nicht nur das verwendete Grundmaterial, sondern auch die Seilkonstruktion berücksichtigt werden.

Es stellen sich somit folgende Probleme:
1. Es ist bisher kein Verfahren zur Erkennung der Ablegereife bekannt, bei dem die tragende Faser selbst Aufschluss darüber gibt, zu welchem Zeitpunkt das Seil abzulegen ist.
2. Die Bruchlängung der Faser ist vom Lastkollektiv (Zugspannung, Temperatur, Zeit) nichtlinear abhängig.
3. Die Bruchlängung eines aus diesen Fasern aufgebauten Seiles ist vom Lastkollektiv (Zugspannung, Temperatur, Zeit) nichtlinear abhängig.
4. Die Bruchlängung des Seils ist unterschiedlich von der Bruchlängung der Faser.

Somit stellt sich die Aufgabe, zur Erkennung der Ablegereife eines Seiles insbesondere aus hochfesten Fasermaterialien, wie z.B. aus UHMWPE-Fasern, ein Verfahren zur Verfügung zu stellen, mit dem die Ablegereife des Seiles unabhängig von der Beanspruchungshistorie bestimmt werden kann.

Diese Aufgabe wird mit einem Verfahren zur Bestimmung der Ablegereife eines Seiles aus textilem Fasermaterial gelöst, wobei im Zuge der Verwendung des Seiles die Längung des Seiles über seine gesamte Länge überwacht wird und das Seil abgelegt wird, wenn die Längung des Seiles über die gesamte Länge einen festgelegten Maximalwert (%) überschreitet, welches dadurch gekennzeichnet ist, dass auch die lokale Längung eines diskreten Seilabschnittes überwacht wird und das Seil abgelegt wird, wenn die lokale Längung des Seilabschnittes einen festgelegten Maximalwert (%) überschreitet, wobei der Maximalwert der lokalen Längung des Seilabschnittes größer ist als der Maximalwert der Längung des Seiles über die gesamte Länge.

### DETAILLIERTE BESCHREIBUNG DER ERFINDUNG

Der Begriff "Seil aus textilem Fasermaterial" bedeutet, dass die wesentlichen Bestandteile des Seiles, insbesondere seine tragenden Elemente aus textilem Fasermaterial, wie z.B. Litzen aus Kunstfasern, bestehen. Das erfindungsgemäße Seil kann auch Bestandteile aus anderen Materialien aufweisen, wie z.B. einen Kern aus nichttextilem Material, einen Mantel aus nichttextilem Material, das Seil oder Seilbestandteile imprägnierende Materialien oder auch einzelne nicht-textile Litzen mit spezieller Funktion, z.B. zur Übertragung elektrischer Signale.

Bevorzugt besteht das Seil, sowohl was tragende als auch nicht-tragende Bestandteile betrifft, aus textilem Fasermaterial.

Es hat sich in der Praxis in Windenanwendungen gezeigt, dass das Verhalten von Seilen aus kriechendem, textilem Fasermaterial (insbesondere von UHMWPE) von den Faserdaten abweicht:
So zeigt sich in Versuchen an einem Kranseilprüfstand (wie z.B. in WO 2012/146380 A beschrieben):
1. Wird ein Seil aus textilem, kriechendem hochfesten Fasermaterial (z.B. UHMWPE) konstanten Lasten mit Sicherheitsfaktoren 100 > Sf > 2,8 bei nicht konstanten Temperaturen unterworfen, so nimmt die irreversible Längung (Kriechlängung) des Seiles über die Zeit zu.
2. Jeder Seilabschnitt kann dabei in Abhängigkeit der Anwendung verschiedenen Belastungen (Seilzugkräfte, Anzahl Biegewechsel je Hub, Belastungsdauer etc.) ausgesetzt sein. Das Seil ist über seine Seillänge somit je nach Seilabschnitt unterschiedlichen Bedingungen ausgesetzt.
3. Es hat sich gezeigt, dass die Versagenslängung des untersuchten textilen Seiles aus UHMWPE in Abhängigkeit von Zugspannung, Temperatur und Einwirkdauer auf einem Kranseilprüfstand in einem vergleichsweise geringen Wertebereich von ca. 1,8% bis 3,8% liegt.

Weiters zeigt sich in Versuchen auf einer Dauerbiegemaschine:
4. Auf Einfachdauerbiegemaschinen, wie sie heute in der Seilforschung Stand der Technik sind, liegt die Bruchlängung eines untersuchten textilen Seiles aus UHMWPE in einem vergleichsweise geringen Wertebereich von ca. 2,5% bis 3,1%.

Es hat sich jedoch gezeigt dass die lokale Versagenslängung in kleinen Seilabschnitten (hier: der Einfachbiegezone) deutlich höher ist als die Bruchlängung des gesamten Seiles. Die lokale Versagenslängung liegt in einem Bereich von 10-16%.

Diese Erkenntnis liegt dem erfindungsgemäßen Verfahren zugrunde, wonach zusätzlich zur Überwachung der Bruchlängung des gesamten Seiles die Überwachung der Bruchlängung kleiner Seilabschnitte herangezogen wird, da diese auf Grund der größeren Veränderung wesentlich genauer zu überwachen ist.

Weiterhin hat sich gezeigt, dass die lokale Versagenslängung kleiner Seilabschnitte nahezu unabhängig von der Zugspannung sowie der Temperatur und nahezu konstant ist. Diese Erkenntnis ist völlig konträr zur heutigen insbesondere von Faserherstellern vertretenen und oben zusammen gefassten Lehrmeinung.

Gemäß dieser Erkenntnis ist somit die Bruchlängung kleiner Seilabschnitte unabhängig von Zugspannung, Temperatur und Einwirkdauer, und es kann daher das Seil anhand von lokal auftretenden absoluten Höchstlängungen überwacht werden.

Erfindungsgemäß wird somit zusätzlich zur Überwachung der Längung des Seiles über seine gesamte Länge die lokale Längung eines oder insbesondere auch mehrerer Seilabschnitte(s) überwacht.

Aufgrund der oben beschriebenen überraschenden Erkenntnis kann dabei der zur Feststellung der Ablegereife festzulegende Maximalwert deutlich oberhalb des Maximalwertes für die Längung über die gesamte Länge des Seiles liegen und somit viel genauer festgestellt werden. Erfindungsgemäß wird das Seil auch dann abgelegt, wenn ein solcher lokaler Maximalwert in einem kleinen Seilabschnitt überschritten wird, selbst wenn der Maximalwert für die Längung über die gesamte Länge des Seiles noch nicht erreicht wird.

Die festzustellende lokale Längung in (%) ist dabei auf die ursprüngliche Länge eines Seilabschnittes mit einer Länge von 10mal der Schlaglänge des Seiles in diesem Seilabschnitt bezogen.

In einer bevorzugten Ausführungsform der vorliegenden Erfindung liegt der Maximalwert (%) der lokalen Längung des diskreten Seilabschnittes um einen Faktor von 1,2 bis 20, bevorzugt 3,0 bis 15, höher als der Maximalwert (%) der Längung des Seiles über die gesamte Länge.

Die jeweiligen Maximalwerte für die Längung des Seiles über die gesamte Länge sowie für die maximal zulässige Längung lokaler Seilabschnitte sind dabei abhängig von der exakten Materialtype des eingesetzten Fasermaterials sowie von der Seilkonstruktion (Schlaglänge, Zwirndrehung usw.) festzulegen.

Beispielsweise wurde für ein Seil aus einem Fasermaterial aus Aramid gefunden, dass der Faktor bei 1,4 liegt. Für ein anderes Seil aus einer bestimmten UHMWPE-Type (Dyneema®) wurde ein Faktor von 10 festgestellt.

Diese Werte sind vor der Verwendung des Seiles im Einsatz experimentell mittels Versuchen auf einem Kranseilprüfstand (z.B. WO 2012/146380A2) sowie Dauerbiegeversuchen (wie aus dem Stand der Technik bekannt) zu bestimmen.

Eine weitere bevorzugte Ausführungsform des erfindungsgemäßen Verfahrens ist dadurch gekennzeichnet, dass das überwachte Seil ein Kern-/Mantelseil ist.

Seile aus einem textilen Fasermaterial, welche in einer Kern/Mantel-Konstruktion vorliegen, sind an sich bekannt, siehe z.B. das Dokument US 2005/011344 A1. Ein solches Seil besteht aus einem oder mehreren beispielsweise geflochtenen oder geschlagenen Kern(en) aus textilem Fasermaterial, welche(r) von einem textilen Fasermaterial als Mantel umhüllt, z.B. umflochten ist bzw. sind.

Für die Zwecke der vorliegenden Erfindung steht im folgenden der Begriff "Kern" sowohl für einen einzigen Kern als auch für eine Mehrzahl von in einem Seil vorhandenen Kernen.

Die Überwachung der Längung des Seiles bzw. des Seilabschnittes kann dabei im Kern des Seiles erfolgen. Dies hat den Vorteil, dass die Längung des tragenden Fasermaterials im Kern einen besseren Aufschluss über den Zustand des Seiles gibt.

Ebenso oder auch zusätzlich kann die Überwachung der Längung des Seiles bzw. des Seilabschnittes im Mantel des Seiles erfolgen.

Es sind auch Kombinationen dieser Maßnahmen möglich, wonach z.B. die Längung diskreter Abschnitte im Kern überwacht wird, während die Längung der Gesamtlänge am Mantel überwacht, und umgekehrt.

Bevorzugt wird als diskreter Seilabschnitt ein Seilabschnitt überwacht, der bei Verwendung des Seiles in einer Biegezone zu liegen kommt. Gerade in solchen Abschnitten besteht eine besondere Beanspruchung des Seiles und kommt daher der Überwachung einer lokalen Längung große Bedeutung zu.

Besonders bevorzugt wird das überwachte Seil als Lastseil, insbesondere mit Treibscheiben- oder Trommelantrieb, verwendet. Insbesondere eignet sich das erfindungsgemäße Verfahren hervorragend zur Bestimmung der Ablegereife von Kranseilen, Aufzugsseilen und Ähnlichem.

Die Bestimmung der Längung des Seiles bzw. des Seilabschnittes kann in an sich bekannter Weise mittels im Seil bzw. im Kern und/oder im Mantel des Seiles vorhandener Indikatorfasern erfolgen.

Alternativ können auch nicht-textile Indikatormaterialien, z.B. ferromagnetische Indikatoren eingesetzt werden.

Bevorzugt wird das erfindungsgemäße Verfahren an einem Seil angewendet, dessen lastttragendes Fasermaterial aus hochfesten Kunststofffasern besteht.

Als "hochfest" werden für die Zwecke der vorliegenden Erfindung Fasern mit einer Zugfestigkeit von mindestens 14 cN/dtex, bevorzugt einer Zugfestigkeit größer 24 cN/dtex, besonders bevorzugt größer 30 cN/dtex, verstanden. Als hochfeste Faserntypen mit entsprechenden Zugfestigkeiten sind z.B. UHMWPE-Fasern (Dyneema®), Aramidfasern, LCP-Fasern und PBO-Fasern bekannt. Bevorzugt sind die hochfesten Kunststofffasern zumindest teilweise, besonders bevorzugt zur Gänze UHMWPE-Fasern.

Unter "lasttragendem Fasermaterial" ist jener Teil des Fasermaterials des Seiles zu verstehen, der zur Aufnahme der bei der Anwendung des Seiles auftretenden Zugkräfte beiträgt.

Das erfindungsgemäße Verfahren wird bevorzugt an einem Seil durchgeführt,
a) dessen lasttragendes Fasermaterial aus hochfesten Kunststofffasern besteht
b) das in Form eines Spirallitzenseiles vorliegt
c) das mindestens zwei, bevorzugt mindestens drei konzentrische lasttragende Litzenlagen aufweist
d) wobei die einzelnen Litzen der Litzenlagen gegeneinander bewegbar sind
e) wobei der Füllungsgrad an textilem Fasermaterial ≥ 75%, bevorzugt ≥ 85% beträgt und
e) wobei die äußerste Schicht des Seiles einen Reibungskoeffizient µ gegenüber Stahl von µ < 0,15 aufweist.

Ein solches Seil ist in der PCT/EP2015/075032 (nicht vorveröffentlicht) beschrieben, auf deren Offenbarung hiermit Bezug genommen wird.

### Beispiele:

### Beispiel 1:

Prüfung eines Seiles mit den oben angeführten Merkmalen a) bis e) aus hochfestem textilem Fasermaterial UHMWPE:
Die Überwachung eines diskreten Seilabschnittes, der in einer Biegezone zu liegen kam, ergab über die Lebensdauer des Seiles eine Längung von 15,5%.

Die Überwachung des gesamten Seiles ergab über die Lebensdauer des Seiles eine Längung von 2,9%.

Somit ergibt sich ein Faktor von 15,5/2,9 = 5,35, um welchen am Ende der Lebensdauer die Längung des diskreten Seilabschnittes höher ist als jene des Seiles über die gesamte Länge.

Zur Bestimmung der Ablegereife eines solchen Seiles wurde als Maximalwert für die lokale Längung entsprechend festgesetzt, dass das Seil unter anderem dann abzulegen ist, wenn die lokale Längung eines diskreten Seilabschnittes einen Wert von 6,0% überschreitet bzw. wenn die Längung des gesamten Seiles 1,6% überschreitet.

### Beispiel 2:

Prüfung eines Seiles mit den oben angeführten Merkmalen a) bis e) aus hochfestem textilem Fasermaterial Aramid:
Die Überwachung eines diskreten Seilabschnittes, der in einer Biegezone zu liegen kam, ergab über die Lebensdauer des Seiles eine Längung von 5,2%.

Die Überwachung des gesamten Seiles ergab über die Lebensdauer des Seiles eine Längung von 3,0%.

Somit ergibt sich ein Faktor von 5,2/3,0 = 1,73, um welchen am Ende der Lebensdauer die lokale Längung des diskreten Seilabschnittes höher ist als jene des Seiles über die gesamte Länge.

Zur Bestimmung der Ablegereife eines solchen Seiles wurde als Maximalwert für die lokale Längung entsprechend festgesetzt, dass das Seil unter anderem dann abzulegen ist, wenn die lokale Längung eines diskreten Seilabschnittes einen Wert von 5,0% überschreitet bzw. wenn die Längung des gesamten Seiles 2,9% überschreitet.

## Patentansprüche

1. Verfahren zur Bestimmung der Ablegereife eines Seiles aus textilem Fasermaterial, **gekennzeichnet durch** die Kombination der Maßnahmen, dass im Zuge der Verwendung des Seiles
- die Längung des Seiles über seine gesamte Länge überwacht wird und das Seil abgelegt wird, wenn die Längung des Seiles über die gesamte Länge einen festgelegten Maximalwert in % überschreitet, und
- die lokale Längung eines diskreten Seilabschnittes überwacht wird und das Seil abgelegt wird, wenn die lokale Längung des Seilabschnittes einen festgelegten Maximalwert in % überschreitet,
wobei der Maximalwert der lokalen Längung des Seilabschnittes größer ist als der Maximalwert der Längung des Seiles über die gesamte Länge.

2. Verfahren gemäß Anspruch 1, **dadurch gekennzeichnet, dass** der Maximalwert in % der lokalen Längung des diskreten Seilabschnittes um einen Faktor von 1,2 bis 20, bevorzugt 3,0 bis 15 höher liegt als der Maximalwert in % der Längung des Seiles über die gesamte Länge.

3. Verfahren gemäß Anspruch 1 oder 2, **dadurch gekennzeichnet, dass** es an einem Seil angewendet wird, welches ein Kern-/Mantelseil ist.

4. Verfahren gemäß Anspruch 3, **dadurch gekennzeichnet, dass** die Überwachung der Längung des Seiles bzw. des Seilabschnittes im Kern des Seiles erfolgt.

5. Verfahren gemäß Anspruch 3 oder 4, **dadurch gekennzeichnet, dass** die Überwachung der Längung des Seiles bzw. des Seilabschnittes im Mantel des Seiles erfolgt.

6. Verfahren gemäß einem der vorhergehenden Ansprüche, **dadurch gekennzeichnet, dass** als diskreter Seilabschnitt ein Seilabschnitt überwacht wird, der bei Verwendung des Seiles in einer Biegezone zu liegen kommt.

7. Verfahren gemäß einem der vorhergehenden Ansprüche, **dadurch gekennzeichnet, dass** die Verwendung des Seiles eine Verwendung als Lastseil, insbesondere mit Treibscheiben- oder Trommelantrieb, ist.

8. Verfahren gemäß einem der vorhergehenden Ansprüche, **dadurch gekennzeichnet, dass** die Bestimmung der Längung des Seiles bzw. des Seilabschnittes mittels im Seil vorhandener Indikatorfasern erfolgt.

9. Verfahren gemäß einem der vorhergehenden Ansprüche, **dadurch gekennzeichnet, dass** es an einem Seil angewendet wird, welches, sowohl was tragende als auch nicht-tragende Bestandteile betrifft, im Wesentlichen aus textilem Fasermaterial besteht.

10. Verfahren gemäß einem der vorhergehenden Ansprüche, **dadurch gekennzeichnet, dass** es an einem Seil angewendet wird, dessen lastttragendes Fasermaterial aus hochfesten Kunststofffasern besteht.

11. Verfahren gemäß Anspruch 10, **dadurch gekennzeichnet, dass** die hochfesten Kunststofffasern zumindest teilweise UHMWPE-Fasern sind.

12. Verfahren gemäß Anspruch 10 oder 11, **dadurch gekennzeichnet, dass** es an einem Seil durchgeführt wird,
a) dessen lasttragendes Fasermaterial aus hochfesten Kunststofffasern besteht
b) das in Form eines Spirallitzenseiles vorliegt
c) das mindestens zwei, bevorzugt mindestens drei konzentrische lasttragende Litzenlagen aufweist
d) wobei die einzelnen Litzen der Litzenlagen gegeneinander bewegbar sind
e) wobei der Füllungsgrad an textilem Fasermaterial ≥ 75%, bevorzugt ≥ 85% beträgt und
e) wobei die äußerste Schicht des Seiles einen Reibungskoeffizient µ gegenüber Stahl von µ < 0,15 aufweist.

## Claims

1. A method of determining the replacement state of wear of a rope made of a textile fibre material, **characterized by** the combination of the measures that, in the course of using the rope,
- the elongation of the rope is monitored across its entire length and the rope is discarded when the elongation of the rope across the entire length exceeds a specified maximum value in %, and
- the local elongation of a discrete rope section is monitored and the rope is discarded when the local elongation of the rope section exceeds a specified maximum value in %,
wherein the maximum value of the local elongation of the rope section is greater than the maximum value of the elongation of the rope across the entire length.

2. A method according to claim 1, **characterized in that** the maximum value in % of the local elongation of the discrete rope section is higher by a factor of 1.2 to 20, preferably 3.0 to 15, than the maximum value in % of the elongation of the rope across the entire length.

3. A method according to claim 1 or 2, **characterized in that** it is applied to a rope which is a core/sheath rope.

4. A method according to claim 3, **characterized in that** the monitoring of the elongation of the rope or, respectively, the rope section takes place in the core of the rope.

5. A method according to claim 3 or 4, **characterized in that** the monitoring of the elongation of the rope or, respectively, the rope section takes place in the sheath of the rope.

6. A method according to any of the preceding claims, **characterized in that**, as a discrete rope section, a rope section is monitored which comes to lie in a bending zone when the rope is being used.

7. A method according to any of the preceding claims, **characterized in that** the use of the rope is a use as a load rope, in particular with a traction sheave or drum drive.

8. A method according to any of the preceding claims, **characterized in that** the determination of the elongation of the rope or, respectively, the rope section is effected by means of indicator fibres provided in the rope.

9. A method according to any of the preceding claims, **characterized in that** it is applied to a rope which is essentially made of a textile fibre material with regard to both load-bearing and non-load-bearing components.

10. A method according to any of the preceding claims, **characterized in that** it is applied to a rope the load-bearing fibre material of which consists of high-strength plastic fibres.

11. A method according to claim 10, **characterized in that** the high-strength plastic fibres are UHMWPE fibres at least partly.

12. A method according to claim 10 or 11, **characterized in that** it is carried out on a rope
a) the load-bearing fibre material of which consists of high-strength plastic fibres
b) which is provided in the form of a spiral stranded rope
c) which comprises at least two, preferably at least three concentric load-bearing strand layers
d) wherein the individual strands of the strand layers are movable relative to each other
e) wherein the degree of filling with textile fibre material is ≥ 75%, preferably ≥ 85%, and
e) wherein the outermost layer of the rope has a coefficient of friction µ relative to steel of µ < 0.15.

## Revendications

1. Procédé de détermination d'un état d'usure nécessitant le remplacement d'un câble en matériau textile fibreux, **caractérisé par** la combinaison des mesures suivantes au cours de l'utilisation du câble :
- l'allongement du câble sur toute sa longueur est surveillé et le câble est remplacé quand l'allongement du câble sur toute sa longueur dépasse une valeur maximale déterminée en pourcentage et
- l'allongement local d'un segment de câble discret est surveillé et le câble est remplacé quand l'allongement local du segment de câble dépasse une valeur maximale déterminée en pourcentage,
la valeur maximale de l'allongement local du segment de câble est supérieure à la valeur maximale de l'allongement du câble sur toute sa longueur.

2. Procédé selon la revendication 1, **caractérisé en ce que** la valeur maximale en pourcentage de l'allongement local du segment de câble discret est supérieur d'un facteur de 1,2 à 20, de préférence de 3,0 à 15, à la valeur maximale en pourcentage de l'allongement du câble sur toute sa longueur.

3. Procédé selon la revendication 1 ou 2, **caractérisé en ce qu'**il est appliqué à un câble ayant une âme et un manteau.

4. Procédé selon la revendication 3, **caractérisé en ce que** la surveillance de l'allongement du câble ou du segment de câble a lieu au niveau de l'âme du câble.

5. Procédé selon la revendication 3 ou 4, **caractérisé en ce que** la surveillance de l'allongement du câble ou du segment de câble a lieu au niveau de la gaine du câble.

6. Procédé selon l'une des revendications précédentes, **caractérisé en ce qu'**en guise de segment de câble discret est surveillé un segment de câble qui est situé dans une zone de courbure au cours de l'utilisation du câble.

7. Procédé selon l'une des revendications précédentes, **caractérisé en ce que** le câble est utilisé comme câble de charge, en particulier avec un entraînement à poulie ou à tambour.

8. Procédé selon l'une des revendications précédentes, **caractérisé en ce que** la détermination de l'allongement du câble ou du segment de câble se fait au moyen de fibres témoins présentes dans le câble.

9. Procédé selon l'une des revendications précédentes, **caractérisé en ce qu'**il est appliqué à un câble qui est constitué essentiellement d'un matériau textile fibreux, en ce qui concerne aussi bien les éléments porteurs que non porteurs.

10. Procédé selon l'une des revendications précédentes, **caractérisé en ce qu'**il est appliqué à un câble dont le matériau textile porteur est constitué de fibres de plastique à haute résistance.

11. Procédé selon la revendication 10, **caractérisé en ce que** les fibres de plastique à haute résistance sont au moins en partie des fibres en UHMWPE.

12. Procédé selon la revendication 10 ou 11, **caractérisé en ce qu'**il est appliqué à un câble
a) dont le matériau fibreux porteur est constitué de fibres de plastique à haute résistance,
b) qui se présente sous la forme d'un câble à torons spiralé,
c) qui possède au moins deux, de préférence trois couches de torons porteuses concentriques,
d) les différents torons des couches de torons étant mobiles entre eux,
e) le taux de remplissage en matériau textile fibreux étant ≥ 75 %, de préférence ≥ 85 % et
e) la couche la plus extérieure du câble possédant un coefficient de frottement µ par rapport à l'acier de µ < 0,15.
